# NEUE EUROPÄISCHE PATENTSCHRIFT

(11) **EP 0 921 117 B2**
(45) Veröffentlichungstag und Bekanntmachung des Hinweises auf die Entscheidung über den Einspruch: **07.01.2009**
(45) Hinweis auf die Patenterteilung: 10.12.2003
(21) Anmeldenummer: 99102583.4
(22) Anmeldetag: 18.08.1994
(51) Int. Cl.: C07C 279/20, C07C 279/22, C07D 295/155, C07D 231/12, C07D 207/327, C07C 311/59

(54) **Acylguanidine**
Acylguanidines
Acylguanidines

(30) Priorität: 24.08.1993 DE 4328352
(43) Veröffentlichungstag der Anmeldung: 09.06.1999
(62) Teilanmeldung aus: 94112895.1
(73) Patentinhaber: Sanofi-Aventis Deutschland GmbH, 65929 Frankfurt am Main (DE)
(72) Erfinder: Kleemann, Heinz-Werner Dr., 65474 Bischofsheim (DE); Lang, Hans-Jochen Dr., 65719 Hofheim (DE); Weichert, Andreas Dr., 63329 Egelsbach (DE); Crause, Peter Dr., 65719 Hofheim (DE); Scholz, Wolfgang Dr., 65760 Eschborn (DE); Albus, Udo Dr., 61197 Florstadt (DE); Schwark, Jan-Robert Dr., 65779 Kelkheim (DE)

(56) Entgegenhaltungen:
- EP-A- 0 416 499
- EP-A- 0 556 673
- EP-A- 0 589 336
- EP-A- 0 604 852
- WO-A-84/00875
- CA-A- 2 089 440
- BEYER, WALTER: 'Lehrbuch der organischen Chemie', Bd. 21, 1988, S. HIRZEL VERLAG, STUTTGART, DE Artikel W. WALTER: 'Heterocyclische Verbindungen', Seiten 703 - 704
- BEYER, WALTER: 'Lehrbuch der organischen Chemie', Bd. 21, 1988, S. HIRZEL VERLAG, STUTTGART, DE Artikel W. WALTER: 'Heterocyclische Verbindungen', Seite 709

## Beschreibung

Die Erfindung betrifft Guanidin-Verbindungen der Formel III 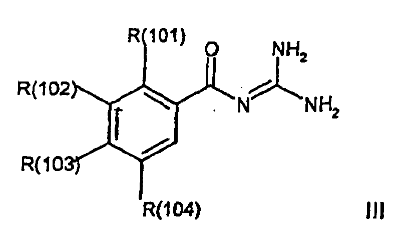 worin bedeuten:
R(101)
F, Cl, CH₃ oder CF₃;
R(102)
Wasserstoff, F, Cl, Br, -CN, R(13)-SO₂, R(14)R(15)N-CO-, R(16)-CO- oder R(17)R(18)N-SO₂-;
R(13), R(14), R(16) und R(17) unabhängig
(C₁-C₈)-Alkyl, (C₃-C₄)-Alkenyl, -CₙH₂ₙ-R(20) oder CF₃;
n Null oder 1;
R(20)
(C₃-C₆)-Cycloalkyl oder Phenyl,
welches nicht substituiert ist oder substituiert mit 1-2 Substituenten aus der Gruppe F, Cl, CF₃, Methyl, Methoxy und NR(21)R(22);
R(21) und R(22)
Wasserstoff oder Methyl;
wobei R(14), R(16) und R(17) auch in der Bedeutung von Wasserstoff stehen,
R(15) und R(18) unabhängig
Wasserstoff oder Methyl;
R(103)
(C₁-C₉)-Heteroaryl,
das über C oder N verknüpft ist und das unsubstituiert oder substituiert ist mit 1 - 2 Substituenten aus der Gruppe bestehend aus F, Cl, CF₃, CH₃, Methoxy, Hydroxy, Amino, Methylamino und Dimethylamino;
oder
R(103)
R(72)-SO₂ oder R(73)R(74)N-SO₂-;
R(72)
(C₁-C₄)-Alkyl, CF₃, (C₃-C₄)-Alkenyl oder -CₛH₂ₛ-R(75);
s Null oder 1;
R(75)
(C₃-C₆)-Cycloalkyl oder Phenyl;
welches nicht substituiert oder substituiert ist mit 1-2 Substituenten aus der Gruppe F, Cl, CF₃, Methyl, Methoxy und NR(76)R(77);
R(76) und R(77)
Wasserstoff oder CH₃;
R(73)
Wasserstoff, (C₁-C₄)-Alkyl, CF₃, (C₃-C₄)-Alkenyl oder -C_{w}H_{2w}-R(78);
w Null oder 1;
R(78)
(C₃-C₆)-Cycloalkyl oder Phenyl,
welches nicht substituiert oder substituiert ist mit 1-2 Substituenten aus der Gruppe F, Cl, CF₃, Methyl, Methoxy und NR(79)R(80);
R(79) und R(80)
Wasserstoff oder CH₃;
R(74) Wasserstoff oder CH₃;
wobei R(73) und R(74) gemeinsam 4 oder 5 Methylengruppen sein können, von denen eine CH₂-Gruppe durch Sauerstoff, S, NH, N-CH₃ oder N-Benzyl ersetzt sein kann;
R(104)
R(87)-SO₂;
R(87)
(C₁-C₄)-Alkyl oder CF₃.

In der Literatur sind bereits einige Benzoylguanidine bekannt. Die beiden Patentanmeldungen EP416499 (CA2089440) und EP556673 beschreiben Benzoylguanidine, jedoch mit einem anderen Substitutionsmuster als die erfindungsgemäßen Verbindungen.

Die erfindungsgemäßen Verbindungen III werden vorteilhaft verwendet zum Herstellen von diacylsubstituierten Guanidinen der Formel I 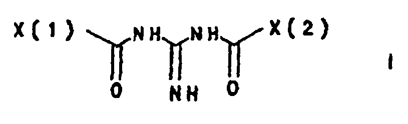 worin bedeuten:
X(1) und X(2) 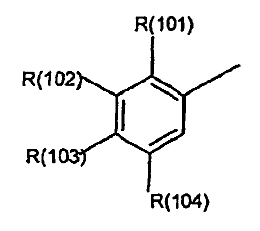 R(101)
F, Cl, CH₃ oder CF₃;
R(102)
Wasserstoff, F, Cl, Br, -CN, R(13)-SO₂, R(14)R(15)N-CO-, R(16)-CO- oder R(17)R(18)N-SO₂-;
R(13), R(14), R(16) und R(17) unabhängig
(C₁-C₈)-Alkyl, (C₃-C₄)-Alkenyl, -CₙH₂ₙ-R(20) oder CF₃;
n Null oder 1;
R(20)
(C₃-C₆)-Cycloalkyl oder Phenyl,
welches nicht substituiert ist oder substituiert mit 1-2 Substituenten aus der Gruppe F, Cl, CF₃, Methyl, Methoxy und NR(21)R(22);
R(21) und R(22)
Wasserstoff oder Methyl;
wobei R(14), R(16) und R(17) auch in der Bedeutung von Wasserstoff stehen,
R(15) und R(18) unabhängig
Wasserstoff oder Methyl;
R(103)
(C₁-C₉)-Heteroaryl,
das über C oder N verknüpft ist und das unsubstituiert oder substituiert ist mit 1 - 2 Substituenten aus der Gruppe bestehend aus F, Cl, CF₃, CH₃, Methoxy, Hydroxy, Amino, Methylamino und Dimethylamino;
oder
R(103)
R(72)-SO₂ oder R(73)R(74)N-SO₂-;
R(72)
(C₁-C₄)-Alkyl, CF₃, (C₃-C₄)-Alkenyl oder -CₛH₂ₛ-R(75);
s Null oder 1;
R(75)
(C₃-C₆)-Cycloalkyl oder Phenyl;
welches nicht substituiert oder substituiert ist mit 1-2 Substituenten aus der Gruppe F, Cl, CF₃, Methyl, Methoxy und NR(76)R(77); R(76) und R(77)
Wasserstoff oder CH₃;
R(73)
Wasserstoff, (C₁-C₄)-Alkyl, CF₃, (C₃-C₄)-Alkenyl oder -C_{w}H_{2w}-R(78);
w Null oder 1;
R(78)
(C₃-C₆)-Cycloalkyl oder Phenyl,
welches nicht substituiert oder substituiert ist mit 1-2 Substituenten aus der Gruppe F, Cl, CF₃, Methyl, Methoxy und NR(79)R(80);
R(79) und R(80)
Wasserstoff oder CH₃;
R(74) Wasserstoff oder CH₃;
wobei R(73) und R(74) gemeinsam 4 oder 5 Methylengruppen sein können, von denen eine CH₂-Gruppe durch Sauerstoff, S, NH, N-CH₃ oder N-Benzyl ersetzt sein kann;
R(104)
R(87)-SO₂;
R(87)
(C₁-C₄)-Alkyl oder CF₃.
sowie deren pharmazeutisch verträgliche Salze,

Bevorzugt werden Verbindungen der Formel I hergestellt, in denen X(1) gleich X(2) ist, und in denen die anderen Substituenten wie oben definiert sind.

Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(13) bis R(104) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkyl- und Perfluoralkyl-reste können sowohl geradkettig wie verzweigt vorliegen.

Die Verbindungen III werden verwendet in einem Verfahren zur Herstellung der Verbindungen I, dadurch gekennzeichnet, daß man eine Verbindung der Formel II 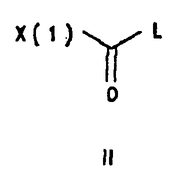 mit einer Verbindung der Formel III 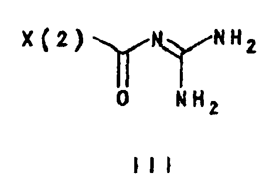 unter Beteiligung einer Base, zum Beispiel K₂CO₃, NaOH oder Triethylamin, umsetzt, worin X(1) und X(2) die angegebene Bedeutung besitzen und L für eine leicht nucleophil substituierbare leaving group steht.

Die aktivierten Säurederivate der Formel II, worin Leine Alkoxy-, vorzugsweise eine Methoxygruppe, eine Phenoxygruppe, Phenylthio, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 - 367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäure-derivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die Einführung einiger Substituenten gelingt durch literaturbekannte Methoden des Palladium-vermittelten cross-couplings von Arylhalogeniden bzw. Aryltriflaten mit beispielsweise Organostannanen, Organoboronsäuren, Organoboranen, Organokupfer- bzw. Organozink-Verbindungen oder terminalen Alkinen.
Die erhaltenen Benzoesäuren werden nach einer der oben beschriebenen Verfahrensvarianten zu aktivierten Benzoesäurederivaten der Formel II umgesetzt. Die Verbindungen der Formel II werden zunächst mit Guanidin zu Verbindungen der Formel III überführt, die nach Isolierung durch Umsetzung mit einer weiteren Verbindung der Formel II zu den erfindungsgemäßen Verbindungen der Formel I umgesetzt werden.

Dialkanoylsubstituierte Guanidine der Formel I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind dialkanoylsubstituierte Guanidine, die sowohl direkt den zellulären Natrium-Protonen-Austauscher (Na⁺/H⁺-exchanger oder Na⁺/H⁺-antiporter ) inhibieren, als auch in vivo mit einer Halbwertszeit zwischen 1 Minute und 10 Stunden einen ihrer Acylreste verlieren und so wiederum Monoacylguanidine, wirkungsvolle Inhibitoren des zellulären Natrium-Protonen-Austauscher, freisetzen.

Damit sind Verbindungen der Formel I potente Inhibitoren des zellulären Natrium-Protonen-Austauscher und führen zu einer Verbesserung der Kinetik der zugrundeliegenden Monoacylguanidine. Darüber hinaus führen sie aufgrund ihrer Lipophilie zu gegenüber den Monoacylguanidinen erhöhten ZNS-Konzentration an Wirkstoff in Form von Dialkanoyl- und Monoacyl-guanidinen.

Verbindungen I sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und - hyperplasien, insbesondere bei Prostatahyperplasie bzw. Prostatahypertrophie verwendet werden.

Die Verbindungen I sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg,
vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- Bn: Benzyl
- CH₂Cl₂: Dichlormethan
- DCI: Desorption-Chemicallonisation
- DIP: Diisopropylether
- DME: Dimethoxyethan
- DMF: N,N-Dimethylformamid
- EE: Ethylacetat (EtOAc)
- El: electron impact
- eq.: Äquivalent
- ES: Elektrospray-Ionisation
- Et: Ethyl
- FAB: Fast Atom Bombardment
- HEP: n-Heptan
- Me: Methyl
- MeOH: Methanol
- mp: Schmelzpunkt
- MTB: Methyltertiärbutylether
- Pd/C: Palladium auf Kohle
- Pt/C: Platin auf Kohle
- RT: Raumtemperatur
- THF: Tetrahydrofuran
- ZNS: Zentralnervensystem

### Experimenteller Teil

Allgemeine Vorschrift zur Herstellung von Monoacyl-guanidinen (III)
Variante A: aus Carbonsäuren (II, L = OH)

1.0 eq. des Carbonsäurederivates der Formel II löst bzw. suspendiert man in wasserfreiem THF ( 5 ml/mmol ) und versetzt sodann mit 1.1 eq.
Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck im Rotationsverdampfer ab, versetzt mit Wasser, stellt mit 2 N HCL auf pH 6 bis 7 und filtriert das entsprechende Monoacyl-guanidin (Formel III) ab. Die so erhaltenen Monoacyl-guanidine können durch Behandeln mit wäßriger, methanolischer oder etherischer Salzsäure oder anderen pharmakologisch verträglichen Säuren in die entsprechenden Salze übergeführt werden.

Allgemeine Vorschrift zur Herstellung von Monoacyl-guanidinen (III)
Variante B: aus Carbonsäure-alkylestern (II, L = O-Alkyl)
1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck im Rotationsverdampfer abdestilliert, dann wird in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

Allgemeine Vorschrift zur Darstellung von Dialkanoylguanidinen (I)
Variante F: aus Carbonsäuren (II, L = OH)

2.0 eq. des Carbonsäurederivates der Formel II löst bzw. suspendiert man in wasserfreiem THF ( 5 ml/mmol ) und versetzt sodann mit 2.2 eq. Carbonyldiimidazol. Nach dem Rühren über 2 Stunden bei RT werden 1.0 eq. Guanidin in die Reaktionslösung eingetragen. Nach dem Rühren über Nacht destilliert man das THF unter vermindertem Druck im Rotationsverdampfer ab, versetzt mit Wasser, stellt mit 2 N HCl auf pH 6 bis 7 und filtriert das entsprechende Dialkanoyl-guanidin (Formel I) ab. Die so erhaltenen Monoacyl-guanidine können durch Behandeln mit wäßriger, methanolischer oder etherischer Salzsäure oder anderen pharmakologisch verträglichen Säuren in die entsprechenden Salze überführt werden.

Allgemeine Vorschrift zur Darstellung von Dialkanoylguanidinen (I)
Variante G: aus Carbonsäureestern (II, L = O-Alkyl)

2.0 eq. des Carbonsäure-alkylesters der Formel II sowie 1.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck im Rotationsverdampfer abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

Allgemeine Vorschrift zur Herstellung von Dialkanoyl-guanidinen (I)
Variante K: aus Monoacyl-guanidinen (III) und einem Carbonsäurederivat der Formel II ( zum Beispiel Carbonsäureester oder aktivierte Carbonsäure)

1.0 eq. des Monoacyl-guanidins der Formel III ( freie Base) sowie 1.0 eq. des Carbonsäurederivats der Formel II (zur Darstellung einer aktivierten Carbonsäure vergleiche Variante A) werden in Isopropanol oder in THF gelöst bzw. suspendiert und bis zum vollständigen Umsatz (Dünnschichtkontrolle) bei passender Temperatur (RT bis Rückfluß) gerührt. Das Lösungsmittel wird unter vermindertem Druck im Rotationsverdampfer abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 10 : 1 chromatographiert.
(Salzbildung vergleiche Variante A)

### Beispiel 1

### Bis-(3-Methylsulfonyl-4-i-propyl-benzoyl)-guanidin

a) 3-Methylsulfonyl-4-i-propyl-benzoylchlorid
   4.0 g 3-Methylsulfonyl-4-i-propyl-benzoesäure, 1.5 ml Thionylchlorid und 3 Tropfen DMF werden in 30 ml Toluol 10 h unter Rückfluß erhitzt. Anschließend wird das Solvens im Vakuum entfernt und das Produkt ohne Reinigung weiter eingesetzt.
b) Bis-(3-Methylsulfonyl-4-i-propyl-benzoyl)-guanidin
   Das Säurechlorid 1 a) und 3.9 g 3-Methylsulfonyl-4-i-propyl-benzoylguanidin werden in 50 ml DMF gelöst, und dann werden 3.4 g K₂CO₃ zugegeben. 4 h wird bei RT gerührt und über Nacht stehen gelassen. Die Lösung wird anschließend eingeengt und der Rückstand in 200 ml Wasser verrührt. Der Feststoff wird abfiltriert und mit 100 ml Wasser gewaschen. Nun wird dieser Feststoff in 100 ml EE gelöst und 1 x mit 10 ml 1 N HCI und anschließend 1x mit 50 ml NaCI-Lösung gewaschen. Über Na₂SO₄ wird getrocknet und das Solvens im Vakuum entfernt. Man erhält einen gelben Feststoff , der durch zweimaliges Verreiben mit 20 ml Diethylether und anschließendes Abfiltrieren gereinigt wird. Das Produkt wird im Vakuum getrocknet und man erhält 1.9 g eines weißen Feststoffs, mp 218 - 221 °C.
   R_{f} (CH₂Cl₂/MeOH 20 1) = 0.57 MS (ES) : 508 (M+1)

### Vorstufen

4-Isopropyl-3-methylsulfonyl-benzoylguanidin-methansulfonat:
Farblose Kristalle, mp 226 - 28°C,

### Syntheseweg:

a) 4-Isopropyl-benzoesäure durch Oxidation von 4-lsopropyl-benzaldehyd mit Natriumperborat in Essigsäure bei 50°C, mp. 118°C.
b) 4-Isopropyl-3-chlorsulfonyl-benzoesäure aus a) durch Erhitzen in Chlorschwefelsäure bei 95°C für 3 h, mp 203 - 4°C,
c) 2-Isopropyl-5-carboxy-benzolsulfinsäure aus b) durch Reduktion mit Natriumsulfit bei 60°C in wäßriger Natronlauge (pH ∼ 9 10), mp 205 - 7°C,
d) 4-Isopropyl-3-methylsulfonyl-benzoesäure aus c) durch Alkylierung mit Methylbromid in Gegenwart von NaOH in DMF bei 60°C für 3 h, mp 209 - 11°C,
e) 4-Isopropyl-3-methylsulfonyl-benzoylguanidin-methansulfonat aus d) durch Reaktion mit Thionylchlorid in Toluol (Rückfluß) für 1 h. Nach Abziehen des Toluols wird in THF aufgenommen und das entstandene Säurechlorid zu einem Gemisch aus Guanidin-hydrochlorid, 2 N NaOH und THF gegeben. Nach 4 h Rühren bei 30-40°C wird das THF abdestilliert, wobei das Produkt als freie Base kristallin anfällt. Anschließende Behandlung mit Methansulfonsäure liefert das Salz.

### Beispiel 2

### Bis-(4-Fluor-3-trifluormethyl-benzoyl)-guanidin

a) 4-Fluor-3-trifluormethyl-benzoylchlorid
   1.5 g 4-Fluor-3-trifluormethyl-benzoesäure, 0.65 ml Thionylchlorid und zwei Tropfen DMF werden in 17 ml Toluol 12 h unter Rückfluß erhitzt, anschließend konzentriert und ohne weitere Reinigung eingesetzt.
b) Bis-(4-Fluor-3-trifluormethyl-benzoyl)-guanidin

Das Säurechlorid 2a) und 0.87 g 4-Fluor-3-trifluormethyl-benzoylguanidin werden in 15 ml DMF gelöst, und dann werden 1.3 g Kaliumcarbonat zugesetzt. Es wird 20 h bei RT gerührt und wie unter 1b) beschrieben aufgearbeitet. Das erhaltene, klare Öl wird säulenchromatographisch gereinigt (Kieselgel, Heptan/Essigester 8:2) und man erhält einen farblosen Feststoff, mp 143-45°C.
R_{f} (Heptan/Essigester 7:3) = 0.7; MS (ES) : 440 (M+1)

### Vorstufe

4-Fluor-3-trifluormethyl-benzoylguanidin:
Farblose Kristalle, mp 159-60°C

### Syntheseweg:

### 4-Fluor-3-trifluormethyl-benzoylguanidin aus 4-Fluor-3-trifluormethyl-benzoesäure durch Reaktion mit N,N'-Carbonyldiimidazol in THF bei RT und anschließende Zugabe von Guanidin

### Beispiel 3

### Bis-[4-(1-Imidazolyl)-3-trifluormethyl-benzoyl]-guanidin

und

### Beispiel 4

### N-[4-(1-Imidazolyl)-3-trifluormethyl-benzoyl]-N'-(4-Fluor-3-trifluormethyl-benzoyl)-guanidin-hydrochlorid

0.25 g Bis-(4-Fluor-3-trifluormethyl-benzoyl)-guanidin, 0.16 g Imidazol und 0.16 g Kaliumcarbonat werden in 5 ml DMF für 16 h erhitzt und danach das Solvens evaporiert. Säulenchromatische Trennung des Rohprodukts ergab 0.12 g Bis-[4-(1-Imidazolyl)-3-trifluormethyl-benzoyl]-guanidin als farblosen Feststoff, mp 130°C, dec., R_{f}(CH₂Cl₂/MeOH 9:1) = 0.4 MS (ES) : 536 (M+1) und 0.07 g N-[4-(1-Imidazolyl)-3-trifluormethyl-benzoyl]-N'-(4-Fluor-3-trifluormethyl-benzoyl)-guanidin als Öl. Behandlung mit HCl_{g}/Ether ergab 0.06 g N-[4-(1-Imidazolyl)-3-trifluormethylbenzoyl]-N'-(4-Fluor-3-trifluormethyl-benzoyl)-guanidin-hydrochlorid als farblosen Feststoff, mp 217-218, dec., R_{f}(CH₂Cl₂/MeOH 9:1) = 0.53; MS (ES) : 488 (M+1)

### Beispiel 5: N, N'-Bis(-1, 2, 3, 4-tetrahydronaphthalin-2-carbonsäure)guanidid wurde gemäß Variante F aus 1, 2, 3, 4-Tetrahydronaphthalin-2-carbonsäure dargestellt und als freie Base isoliert.

MS (ES): 376 (M+1)

mp 99°C

Wurde ebenfalls gemäß Variante K über das 1, 2, 3, 4-Tetrahydronaphthalin-2-carbonsäure-guanidid [dargestellt gemäß Variante A aus 1, 2, 3, 4-Tetrahydronaphthalin-2-carbonsäure: MS (ES): 218 (M+1)] und 1, 2, 3, 4-Tetrahydronaphthalin-2-carbonsäure dargestellt.

### Beispiel 6: N, N'-Bis[-(E)-2-methyl-zimtsäure]guanidid wurde gemäß Variante F aus (E)-2-Methyl-zimtsäure dargestellt und als Hydrochlorid isoliert.

MS (ES): 348 (M+1)

mp 193°C

### Beispiel 7: N, N'-Bis[-3-(2-trifluormethylphenyl)-propionsäure]guanidid wurde gemäß Variante F aus 3-(2-Trifluormethylphenyl)-propionsäure dargestellt und als Hydrochlorid isoliert.

MS (ES): 460 (M+1)

mp 75°C

### Beispiel 8: N, N'-Bis[-3-(2, 5-difluorphenyl)-2-methyl-propionsäure]guanidid

wurde gemäß Variante F aus 3-(2, 5-difluorphenyl)-2-methyl-propionsäure dargestellt und als Hydrochlorid isoliert.
MS (ES): 424 (M+1)
mp amorph

### Pharmakologische Daten:

### Inhibition des Na⁺/H⁺-Exchangers von Kaninchenerythozyten:

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrozyten via Na⁺/H⁺-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des Na⁺-Ausgangsgehalts der Erythrozyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCI, 150 Sucrose, 0,1 Ouabain, 20 Tris-hydroxymethyl-aminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrozyten nach Inkubation errechnet. Der Amiloridhemmbare Natrium-Influx ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

Ergebnisse zur Inhibition des Na⁺/H⁺-Exchangers:

| IC₅₀(µmol) | Beispiel |
|---|---|
| 10 | 1 |
| 10 | 2 |
| 5 | 3 |
| 3 | 4 |
| 0.3 | 5 |
| 1.1 | 6 |
| 2 | 7 |
| 0.3 | 8 |

Analog zu Beispiel 1 werden erhalten:

| Beispiel | R(1) = R(10) | R(2) = R(9) | R(3) = R(8) | R(4) = R(7) | R(5) = R(6) |
|---|---|---|---|---|---|
| 9 | H | H | Cl | n-BuNH- | H |
| 10 | H | H | 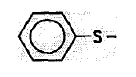 | H₂NSO₂- | H |
| 11 | H | H | 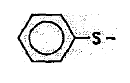 | MeSO₂ | H |
| 12 | H | 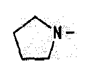 | Me | H | H |
| 13 | H | 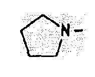 | 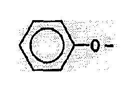 | H | H |
| 14 | H | 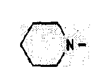 | Me | H | H |
| 15 | H | 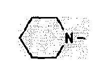 | Cl | H | H |
| 16 | H | 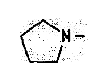 | MeSO₂- | H | H |
| 17 | H | H | NH₂ | MeSO₂ | H |
| 18 | H | H | 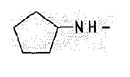 | MeSO₂- | H |
| 19 | H | H | 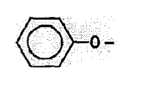 | MeSO₂- | H |
| 20 | H | H | 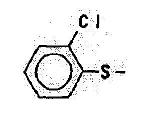 | MeSO₂- | H |
| 21 | H | H | 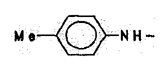 | MeSO₂- | H |
| 22 | H | H | 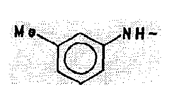 | MeSO₂- | H |
| 23 | H | H | 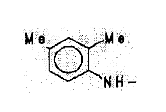 | MeSO₂- | H |
| 24 | H | H | 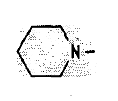 | Cl₂- | H |
| 25 | H | H | (CH₃)₂-CHCH₂-O- | MeSO₂₋ | H |
| 26 | H | H | 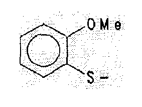 | MeSO₂- | H |
| 27 | H | H | 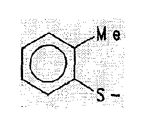 | MeSO₂₋ | H |
| 28 | H | H | 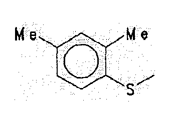 | MeSO₂ | H |
| 29 | H | 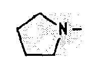 | 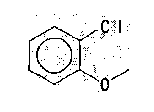 | H | H |
| 30 | H | 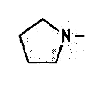 | 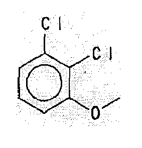 | H | H |
| 31 | H | 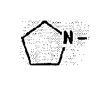 | 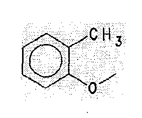 | H | H |
| 32 | H | 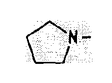 | 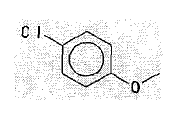 | H | H |
| 33 | H | 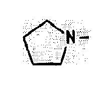 | 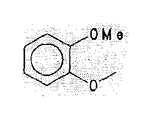 | H | H |
| 34 | H | 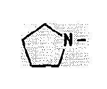 | 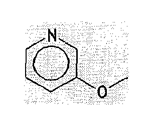 | H | H |
| 35 | H | H | 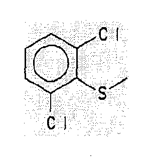 | MeSO₂₋ | H |
| 36 | H | H | 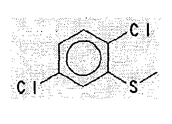 | MeSO₂₋ | H |
| 37 | Me | H | H | Me | H |
| 38 | H | Me | Me | H₂NSO₂- | H |
| 39 | H | H | H | CF₃ | H |
| 40 | H | 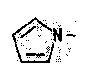 | Cl | H | H |
| 41 | H | H | MeNH- | MeSO₂- | H |
| 42 | H | H | Et₂N- | MeSO₂₋ | H |
| 43 | H | t-Bu | OH | t-Bu | H |
| 44 | H | H | 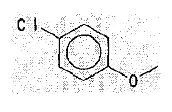 | MeSO₂₋ | H |
| 45 | H | H | 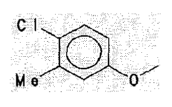 | MeSO₂- | H |
| 46 | H | H | 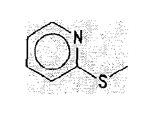 | MeSO₂- | H |
| 47 | H | H | 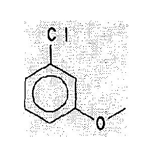 | MeSO₂- | H |
| 48 | H | H | 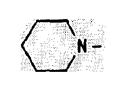 | MeSO₂- | H |
| 49 | H | H | 2-Naphthyl | MeSO₂- | H |
| 50 | H | H | 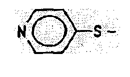 | MeSO₂- | H |
| 51 | H | 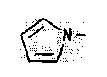 | Me | H | H |
| 52 | H | 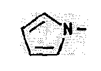 | 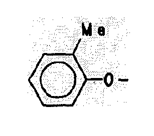 | H | H |
| 53 | H | Cl | Et₂N- | MeSO₂- | H |
| 54 | H | Me₂N- | H | CF₃ | H |
| 55 | H | H | 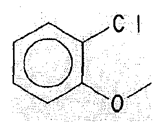 | MeSO₂- | H |
| 56 | H | Br | NH₂ | Br | H |
| 57 | H | Cl | H | CF₃ | H |
| 58 | H | H | 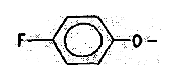 | MeSO₂- | H |
| 59 | H | H | 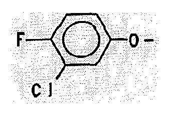 | MeSO₂- | H |
| 60 | H | CF₃ | H | CF₃ | H |
| 61 | H | H | Me | Me | H |
| 62 | H | I | H | CF₃ | H |
| 63 | H | Me | H | Me | H |
| 64 | H | H | t-Bu | H | H |
| 65 | H | H | 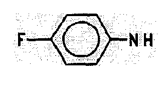 | MeSO₂- | H |
| 66 | H | H | Cl | Me | H |
| 67 | H | H | Me | Br | H |
| 68 | H | H | MeO- | Cl | H |
| 69 | H | Cl | MeO- | Cl | H |
| 70 | H | Br | H | Br | H |
| 71 | H | H | 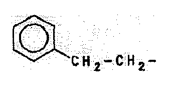 | MeSO₂- | H |
| 72 | H | H | 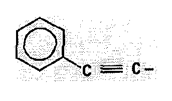 | MeSO₂- | H |
| 73 | NH₂ | Br | H | Me | H |
| 74 | H | N₃ | H | CF₃ | H |
| 75 | H | H | t-Bu | Me₂N- | H |
| 76 | H | H | 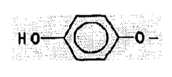 | MeSO₂- | H |
| 77 | H | 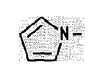 | H | H | H |
| 78 | H | 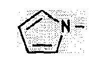 | MeO- | H | H |
| 79 | H | H | Br | Me | H |
| 80 | H | H | F | Cl | H |
| 81 | H | t-Bu | H | t-Bu | H |
| 82 | NH₂ | H | H | Cl | H |
| 83 | H | 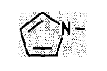 | H | Me₂N | H |
| 84 | H | Me₂N | H | Cl | H |
| 85 | H | H | 7-Isochinolinoxy | MeSO₂- | H |
| 86 | H | H | 6-Chinolinoxy | MeSO₂₋ | H |
| 87 | H | H | 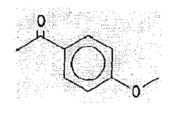 | MeSO₂- | H |
| 88 | H | H | 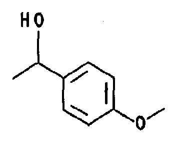 | MeSO₂- | H |
| 89 | H | H | (CH₃)₂CH-CH₂- | MeSO₂- | H |
| 90 | H | H | 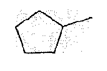 | MeSO₂- | H |
| 91 | H | H | 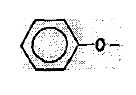 | Me₂N- | H |
| 92 | H | H | 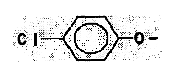 | Me₂N- | H |
| 93 | H | Me | Me₂N- | Me | H |
| 94 | H | H | 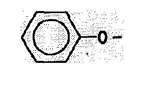 | 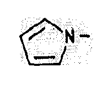 | H |
| 95 | H | Me | 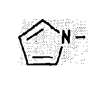 | Me | H |
| 96 | H | H | ClCH=C(CH₃)- | MeSO₂ | H |
| 97 | H | H | i-Pr | Cl | H |
| 98 | H | H | i-Pr | 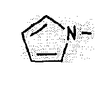 | H |
| 99 | H | H | 5-Chinolinoxy | MeSO₂- | H |
| 100 | H | 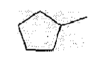 | H | CF₃ | H |
| 101 | H | i-Pr | H | MeSO₂- | H |
| 102 | H | i-P | H | CF₃ | H |
| 103 | H | H | i-Pr | H | H |
| 104 | NH₂ | H | Br | Br | H |
| 105 | H | H | 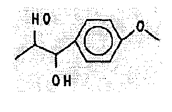 | MeSO₂- | H |
| 106 | H | 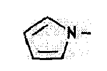 | H | MeSO₂- | H |
| 107 | H | H | 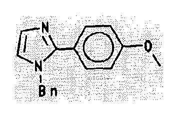 | MeSO₂- | H |
| 108 | H | Cl | 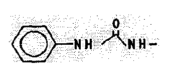 | Cl | H |
| 109 | H | Me₂N | i-Pr | MeSO₂- | H |
| 110 | H | MeHN- | i-Pr | MeSO₂- | H |
| 111 | H | Cl | Cl | Cl | H |
| 112 | H | Cl | H₂N- | Me | H |
| 113 | H | Cl | H₂N | Cl | H |
| 114 | H | H | 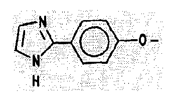 | MeSO₂- | H |
| 115 | H | H | 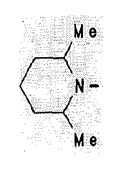 | MeSO₂- | H |
| 116 | H | H | i-Pr | Me₂N- | H |
| 117 | CF₃ | H | H | CF₃ | H |
| 118 | H | Br | H | Me | H |
| 119 | H | Me | Cl | Me | H |
| 120 | H | Me₂N | Me | Br | H |
| 121 | H | CF₃ | H | MeHN- | H |
| 122 | H | H | (CH₃)₂CH-CH₂ | CH₃CO- | H |
| 123 | H | H | 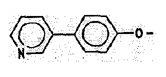 | MeSO₂- | H |
| 124 | H | H | H | CF₃-O- | H |
| 125 | H | Cl | Me₂N | Me | H |
| 126 | H | H | 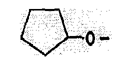 | Cl | H |
| 127 | H | Cl | Me₂N- | Cl | H |
| 128 | H | H | 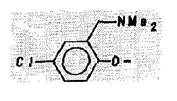 | MeSO₂- | H |
| 129 | H | H | i-Pr | CH₃CO- | H |
| 130 | H | Br | BnO- | CH₃CO- | H |
| 131 | H | H | Br | CF₃ | H |
| 132 | H | H | MeO- | i-Pr | H |
| 133 | H | H | 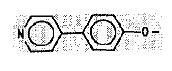 | MeSO₂- | H |
| 134 | H | H | 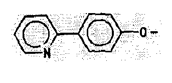 | MeSO₂- | H |
| 135 | H | H | t-Bu | MeO- | H |
| 136 | H | Br | i-Pr | MeSO₂- | H |
| 137 | CF₃ | H | H | H | H |
| 138 | H | H | F | CF₃ | H |
| 139 | H | Ph | H | CF₃ | H |
| 140 | H | H | 1-imidazolyl | CF₃ | H |
| 141 | H | H | Cyclopentyl | CH₃CO- | H |
| 142 | H | H | t-Butylmethyl | CH₃CO- | H |
| 143 | H | H | F | Br | H |
| 144 | H | Br | MeO- | Br | H |
| 145 | NH₂ | Me | H | H | H |
| 146 | H | H | PhO- | CF₃ | H |
| 147 | H | H | Cyclopentyl | CF₃ | H |
| 148 | H | H | Cyclobutyl | MeSO₂- | H |
| 149 | H | Me | H | CF₃ | H |
| 150 | H | H | 4-CF₃-PhO | MeSO₂- | H |
| 151 | H | H | OH | t-Butyl | H |
| 152 | H | Me₂N- | MeO- | Cl | H |
| 153 | H | H | Isopropyl | CF₃ | H |
| 154 | H | CF₃ | H | H | F |
| 155 | H | H | MeO- | t-Butyl | H |
| 156 | H | H | 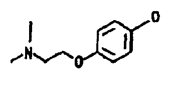 | MeSO₂- | H |
| 157 | H | Br | 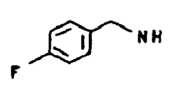 | Br | H |
| 158 | H | H | 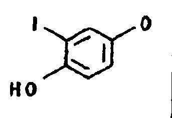 | MeSO2- | H |
| 159 | H | H | 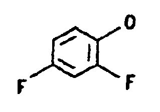 | CH₃CO- | H |
| 160 | H | H | 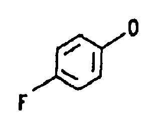 | CH₃CO- | H |
| 161 | H | H | 2-Methylpropyl | t-Butyl | H |
| 162 | H | Cyclopentyl | MeO- | CH₃CO- | H |
| 163 | H | H | Isopropyl | CF₃CF₂ | H |
| 164 | H | H | 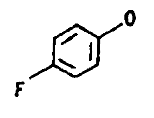 | CF₃SO₂ | H |
| 165 | H | CF₃ | H | H | Cl |
| 166 | H | H | 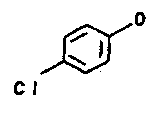 | CF₃ | H |
| 167 | H | H | Ph-C/C | CF₃ | H |
| 168 | H | H | 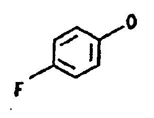 | CF₃ | H |
| 169 | H | F | H | CF₃ | H |
| 170 | H | H | 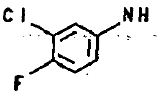 | MeSO₂- | H |
| 171 | H | H | Isopropyl | t-Butyl | H |
| 172 | H | H | n-Butyl | t-Butyl | H |
| 173 | H | H | F | Isopropyl | H |
| 174 | H | H | F | Isobutyl | H |
| 175 | H | I | H | CF3 | F |
| 176 | H | H | CF₃O- | Cl | H |
| 177 | NH₂ | H | Cl | H | Cl |
| 178 | H | H | 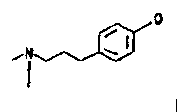 | MeSO₂- | H |
| 179 | H | I | OH | t-Butyl | H |
| 180 | H | H | Isopropyl | CF₃SO₂ | H |
| 181 | H | H | 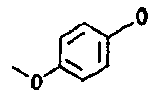 | CF₃ | H |
| 182 | H | H | 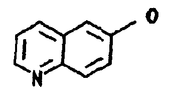 | CF₃ | H |
| 183 | H | H | 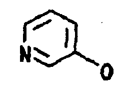 | CF₃ | H |
| 184 | H | H | 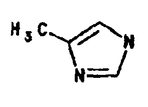 | CF₃ | H |
| 185 | H | H | 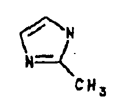 | CF₃ | H |
| 186 | H | H | MeO- | CF₃ | H |
| 187 | H | H | H | Isopropyl | H |
| 188 | H | H | Isopropyl | MeS- | H |

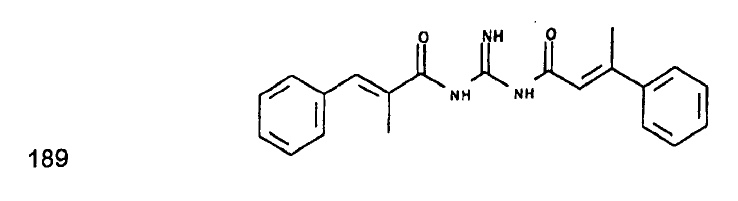

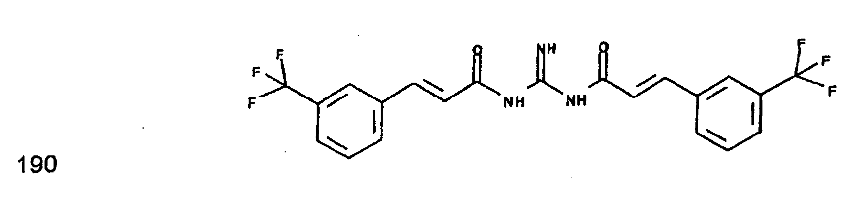

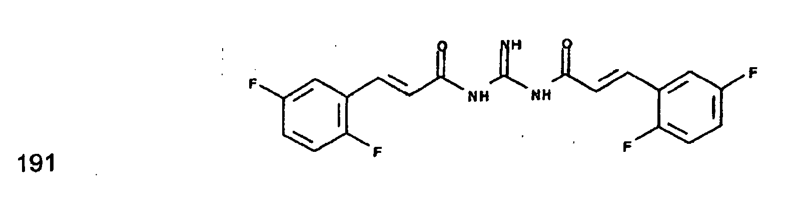

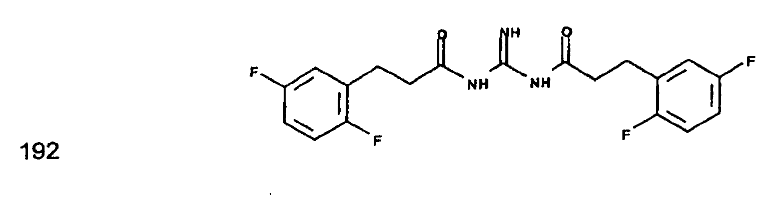

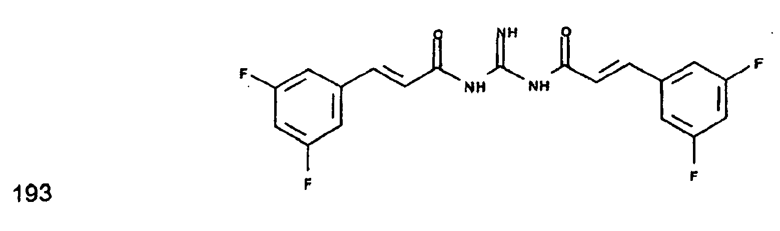

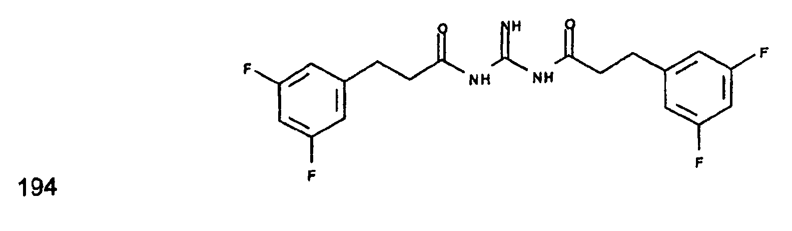

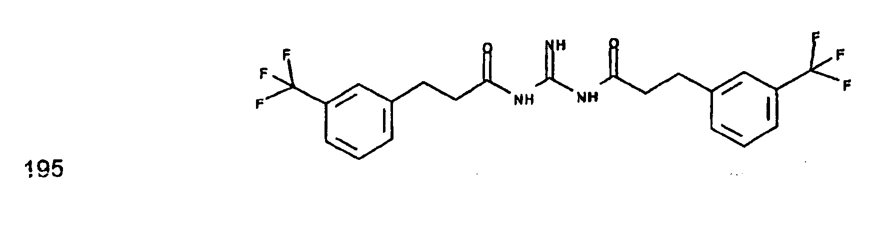

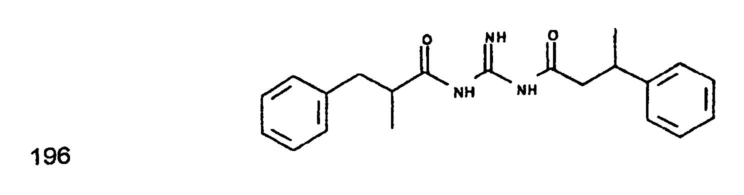

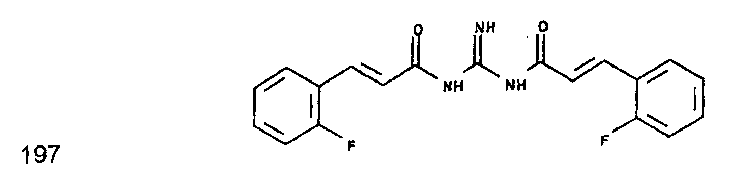

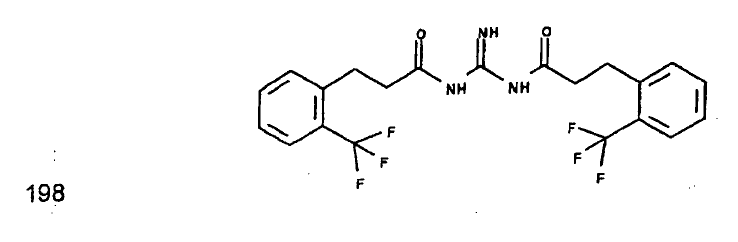

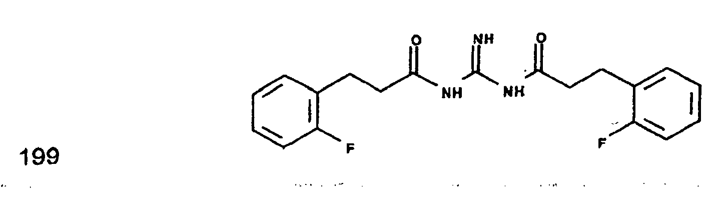

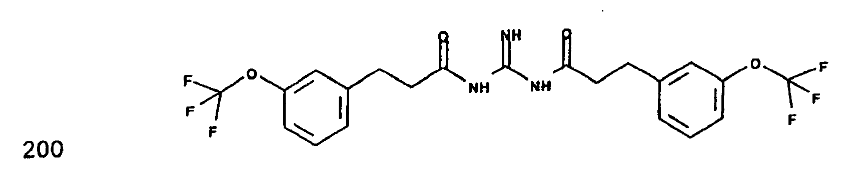

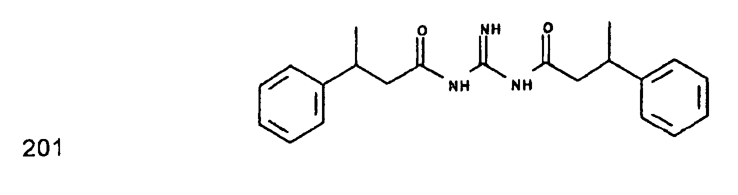

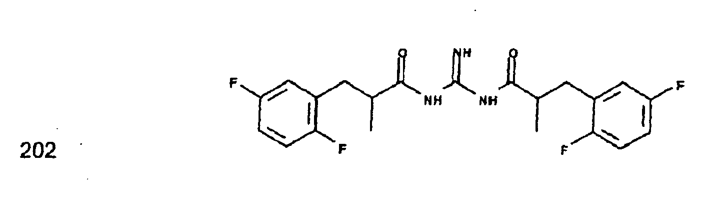

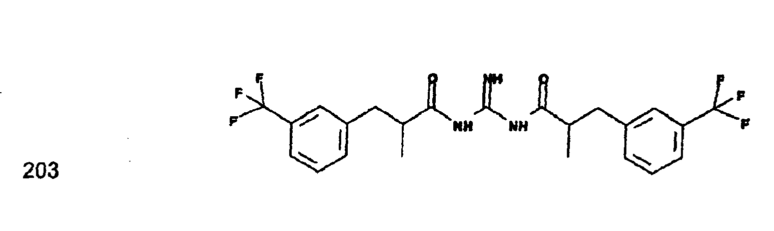

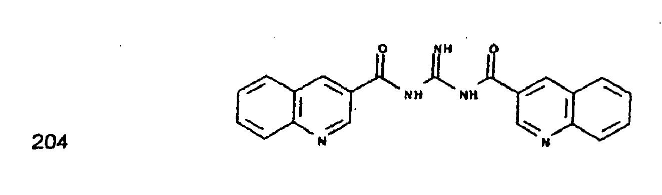

## Patentansprüche

1. Guanidin-Verbindungen der Formel III 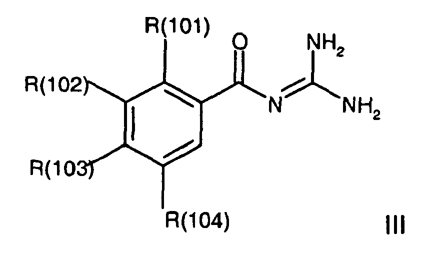 worin bedeuten:
R(101) F, Cl, CH₃ oder CF₃;
R(102) Wasserstoff, F, Cl, Br, -CN, R(13)-SO_{2,} R(14)R(15)N-CO-, R(16)-CO- oder R(17)R(18)N-SO₂-;
R(13), R(14), R(16) und R(17) unabhängig
(C₁-C₈)-Alkyl, (C₃-C₄)-Alkenyl, -CₙH₂ₙ-R(20) oder CF₃;
n Null oder 1;
R(20) (C₃-C₆)-Cycloalkyl oder Phenyl,
welches nicht substituiert ist oder substituiert mit 1 - 2 Substituenten aus der Gruppe F, Cl, CF₃, Methyl, Methoxy und NR(21)R(22);
R(21) und R(22)
Wasserstoff oder Methyl;
wobei R(14), R(16) und R(17) auch in der Bedeutung von Wasserstoff stehen, R(15) und R(18) unabhängig
Wasserstoff oder Methyl;
R(103) (C₁-C₉)-Heteroaryl, das über C oder N verknüpft ist und das unsubstituiert oder substituiert ist mit 1 - 2 Substituenten aus der Gruppe bestehend aus F, Cl, CF₃, CH₃, Methoxy, Hydroxy, Amino, Methylamino und Dimethylamino;
oder
R(103) R(72)-SO₂ oder R(73)R(74)N-SO₂-;
R(72) (C₁-C₄)-Alkyl, CF₃, (C₃-C₄)-Alkenyl oder -CₛH₂ₛ-R(75);
s Null oder 1;
R(75) (C₃-C₆)-Cycloalkyl oder Phenyl;
welches nicht substituiert oder substituiert ist mit 1 - 2 Substituenten aus der Gruppe F, Cl, CF₃, Methyl, Methoxy und NR(76)R(77);
R(76) und R(77)
Wasserstoff oder CH₃;
R(73) Wasserstoff, (C₁-C₄)-Alkyl, CF₃, (C₃-C₄)-Alkenyl oder -C_{w}H_{2w}-R(78);
w Null oder 1;
R(78) (C₃-C₆)-Cycloalkyl oder Phenyl,
welches nicht substituiert oder substituiert ist mit 1 - 2 Substituenten aus der Gruppe F, Cl, CF₃, Methyl, Methoxy und NR(79)R(80); R(79) und R(80)
Wasserstoff oder CH₃;
R(74) Wasserstoff oder CH₃;
wobei R(73) und R(74) gemeinsam 4 oder 5 Methylengruppen sein können, von denen eine CH₂-Gruppe durch Sauerstoff, S, NH, N-CH₃ oder N-Benzyl ersetzt sein kann;
R(104) R(87)-SO₂;
R(87) (C₁-C₄)-Alkyl oder CF₃;

2. Heilmittel, **dadurch gekennzeichnet, dass** es eine wirksame Menge einer Verbindung III nach Anspruch 1 enthält.

## Claims

1. A guanidine compound of the formula III 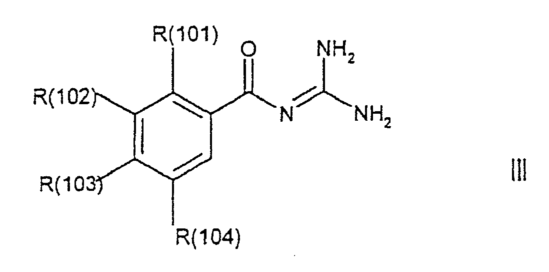 in which:
R(101) is F, Cl, CH₃ or CF₃;
R(102) is hydrogen, F, Cl, Br, -CN,
R(13)-SO₂, R(14)R(15)N-CO-, R(16)-CO- or R(17)R(18)N-SO₂-;
R(13), R(14) , R(16) and R(17)
independently are (C₁-C₈)-alkyl, (C₃-C₄)-alkenyl, -CₙH₂ₙ-R(20) or CF₃;
n is zero or 1;
R(20) is (C₃-C₆)-cycloalkyl or phenyl,
which is not substituted or is substituted by 1-2 substituents from the group consisting of F, C1, CF₃, methyl, methoxy and NR(21)R(22);
R(21) and R(22)
are hydrogen or methyl;
or R(14), R(16) and R(17) are also hydrogen,
R(15) and R(18)
independently are hydrogen or methyl;
R(103) is (C₁-C₉) -heteroaryl
which is linked via C or N and which is unsubstituted or substituted by 1-2 substituents from the group consisting of F, Cl, CF₃, CH₃, methoxy, hydroxyl, amino, methylamino and dimethylamino;
or
R(103) is R(72)-SO₂ or R(73)R(74)N-SO₂-;
R(72) is (C₁-C₄)-alkyl, CF₃, (C₃-C₄)-alkenyl or -CsH₂ₛ-R(75) ;
s is zero or 1;
R(75) is (C₃-C₆)-cycloalkyl or phenyl; which is not substituted or is substituted by 1-2 substituents from the group consisting of F, Cl, CF₃, methyl, methoxy and NR(76)R(77) ;
R(76) and R(77)
are hydrogen or CH₃;
R(73) is hydrogen, (C₁-C₄)-alkyl, CF₃, (C₃-C₄)-alkenyl or -C_{w}H_{2w}-R(78);
w is zero or 1;
R(78) is (C₃-C₆)-cycloalkyl or phenyl,
which is not substituted or is substituted by 1-2 substituents from the group consisting of F, Cl, CF₃, methyl, methoxy and NR(79)R(80) ;
R(79) and R(80)
are hydrogen or CH₃;
R(74) is hydrogen or CH₃;
where R(73) and R(74) together can be 4 or 5 methylene groups,
of which one CH₂ group can be replaced by oxygen, S, NH, N-CH₃ or N-benzyl;
R(104) is R(87)-SO₂;
R(87) is (C₁-C₄)-alkyl or CF₃.

2. A medicine which contains an effective quantity of a compound III as claimed in claim 1.

## Revendications

1. Composés de guanidine de formule III 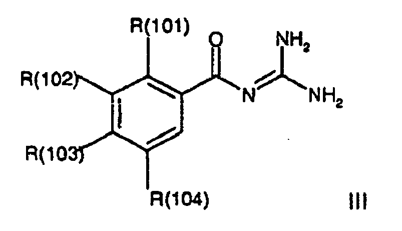 dans laquelle
R(101) signifie F, Cl, CH₃ ou CF₃ ;
R(102) signifie hydrogène, F, Cl, Br, -CN, R(13)-SO₂, R(14)R(15)N-CO-, R(16)-CO- ou R(17)R(18)N-SO₂- ; R(13), R(14) , R(16) et R(17) signifient, indépendamment l'un de l'autre, (C₁-C₈)-alkyle, (C₃-C₄) -alcényle, -CₙH₂ₙ-R (20) ou CF₃ ;
n vaut zéro ou 1 ;
R(20) signifie (C₃-C₆)-cycloalkyle ou phényle, qui est non substitué ou substitué par 1-2 substituants du groupe F, Cl, CF₃, méthyle, méthoxy et NR (21) R (22) ;
R(21) et R(22) signifient hydrogène ou méthyle ;
où R(14), R(16) et R(17) signifient également hydrogène,
R(15) et R(18) signifient indépendamment l'un de l'autre hydrogène ou méthyle ;
R(103) signifie (C₁-C₉) -hétéroaryle,
qui est lié via C ou N et qui est non substitué ou substitué par 1-2 substituants du groupe constituée par F, Cl, CF₃, CH₃, méthoxy, hydroxy, amino, méthylamino et diméthylamino ;
ou
R(103) signifie R(72)-SO₂ ou R(73)R(74)N-SO₂- ;
R72 signifie (C₁-C₄) -alkyle, CF₃, (C₃-C₄) -alcényle ou -CₛH₂ₛ-R(75) ;
s vaut zéro ou 1 ;
R(75) signifie (C₃-C₆)-cycloalkyle ou phényle ; qui est non substitué ou substitué par 1-2 substituants du groupe F, Cl, CF₃, méthyle, méthoxy et NR(76)R(77) ;
R(76) et R(77)
signifient hydrogène ou CH₃ ;
R(73) signifie hydrogène, (C₁-C₄)-alkyle, CF₃, (C₃-C₄) -alcényle ou -C_{w}H_{2w}-R(78) ;
w vaut zéro ou 1 ;
R(78) signifie (C₃-C₆) -cycloalkyle ou phényle, qui est non substitué ou substitué par 1-2 substituants du groupe F, Cl, CF₃, méthyle, méthoxy et NR(79)R(80) ;
R(79) et R(80)
signifient hydrogène ou CH₃ ;
R(74) signifie hydrogène ou CH₃ ;
où R(73) et R(74) peuvent représenter ensemble 4 ou 5 groupes méthylène, dont un groupe CH₂ peut être remplacé par oxygène, S, NH, N-CH₃ ou N-benzyle ;
R (104) signifie R(87)-SO₂ ;
R(87)
signifie (C₁-C₄) -alkyle ou CF₃.

2. Médicament, **caractérisé en ce qu'**il contient une quantité active d'un composé III selon la revendication 1.
